# EUROPEAN PATENT APPLICATION

(11) **EP 4 403 122 A1**
(43) Date of publication of application: **24.07.2024**
(21) Application number: 22952941.7
(22) Date of filing: 28.12.2022
(51) Int. Cl.: A61B 17/32

(54) **ELECTRIC SPIRAL CUTTER FOR ELIMINATING NECK HUMP**

(30) Priority: 25.07.2022 CN 202221922162 U
(71) Applicant: Huang, Xuefeng, Luoyang, Henan 471000 (CN)
(72) Inventor: Huang, Xuefeng, Luoyang, Henan 471000 (CN)
(74) Representative: Sach, Greg Robert
(86) International application number: PCT/CN2022/142575
(87) International publication number: WO 2024/021512

(57) **Abstract**

An electric spiral knife for eliminating neck humps relates to a tool for breaking subcutaneous fiber tissue. A spiral groove (9) arranged clockwise along the side wall of a rod body (11) is installed at one end of the rod body (11); a cutting edge (8) is located at the rear end of a notch of the cross section of the rod body (11) at the spiral groove (9) when the rod body (11) rotates clockwise according to the spiral groove (9) as the front end; a notch at the other side opposite to the cutting edge (8) is set as convex arc transition (10); the rotary driver comprises a motor (3); one end of a flexible shaft (2) is connected with a rotating shaft arranged on the motor (3); and the other end of the flexible shaft (2) is connected with the other end of the rod body (11). In the utility model, the spiral groove is arranged at one end of the rod body and the cutting edge is arranged at the notch of the spiral groove, to achieve the purpose of breaking the fibrous connective tissue in the neck hump by using the cutting edge to more easily suck out the fibrous connective tissue and fat in the neck hump for relieving the disease.

## Description

### Technical Field

The utility model relates to a tool for breaking subcutaneous fiber tissue, in particular to an electric spiral knife for eliminating neck humps.

### Background

It is known that the common medical cosmetic surgical instruments for eliminating neck humps are single-hole probes, top and twin-side bottom probes or porous probes. During operation, a doctor makes a hole in the diseased part of a patient and carries out a liposuction operation on the neck hump on the neck and back by the method of manual liposuction. Because the neck hump not only contains adipose tissue, but also contains a large number of proliferative fibrous connective tissue, the fibrous connective tissue may block a liposuction hole in the process of liposuction and affect the therapeutic effect. Therefore, the purpose of effectively reducing the bulge at the neck hump part of the patient cannot be achieved. The neck hump is easy to relapse because the fibrous connective tissue is not sucked out.

### Summary

In order to overcome the deficiencies in the background, the utility model discloses an electric spiral knife for eliminating neck humps. A spiral groove is arranged at one end of a rod body and a cutting edge is arranged at a notch of the spiral groove, to achieve the purpose of breaking the fibrous connective tissue in the neck hump by using the cutting edge to more easily suck out the fibrous connective tissue and fat in the neck hump for relieving the disease.

In order to achieve the invention purpose, the utility model adopts the following technical solution:

An electric spiral knife for eliminating neck humps comprises a drill bit and a rotary driver; the drill bit comprises a rod body; a spiral groove arranged clockwise along the side wall of the rod body is installed at one end of the rod body; a cutting edge is located at the rear end of a notch of the cross section of the rod body at the spiral groove when the rod body rotates clockwise according to the spiral groove as the front end; a notch at the other side opposite to the cutting edge is set as convex arc transition; the rotary driver comprises a motor; one end of a flexible shaft is connected with a rotating shaft arranged on the motor; and the other end of the flexible shaft is connected with the other end of the rod body.

In the electric spiral knife for eliminating neck humps, a chassis is covered outside the motor.

In the electric spiral knife for eliminating neck humps, a foot switch controls a switch of the motor, a connecting line is arranged between the foot switch and the motor, and the foot switch is connected to a power supply.

In the electric spiral knife for eliminating neck humps, the motor is communicated with one end of the flexible shaft by a speed reducer.

In the electric spiral knife for eliminating neck humps, one spiral groove is arranged on the rod body.

In the electric spiral knife for eliminating neck humps, concave arc transition is set between the cutting edge and the spiral groove.

In the electric spiral knife for eliminating neck humps, a conical end is arranged at the end part of one end of the rod body.

In the electric spiral knife for eliminating neck humps, the rod body is made of metal.

Because the above technical solution is adopted, the utility model has the following beneficial effects:
In the electric spiral knife for eliminating neck humps in the utility model, the spiral groove is arranged at one end of the rod body and the cutting edge is arranged at the notch of the spiral groove, to achieve the purpose of breaking the fibrous connective tissue in the neck hump by using the cutting edge to more easily suck out the fibrous connective tissue and fat in the neck hump for relieving the disease. The utility model has simple and reasonable structure, easy operation, strong practicability and broad market prospects.

### Description of Drawings

Fig. 1 is a structural schematic diagram of the utility model;
Fig. 2 is a structural schematic diagram of a drill bit of the utility model;
Fig. 3 is an A-A view of a rod body in Fig. 2.

In the figures: 1, drill bit; 2, connecting flexible shaft; 3, motor; 4, chassis; 5, connecting line; 6, foot switch; 7, conical end; 8, cutting edge; 9, spiral groove; 10, convex arc transition; 11, rod body; and 12, concave arc transition.

### Detailed Description

The utility model can be explained in more detail through the following embodiments. The utility model is not limited to the following embodiments, and the purpose of the disclosed utility model is to protect all changes and improvements within the scope of the utility model.

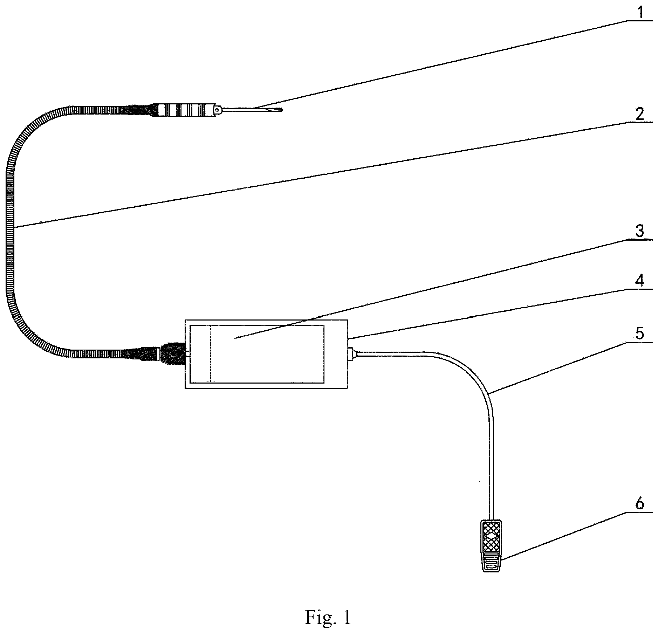

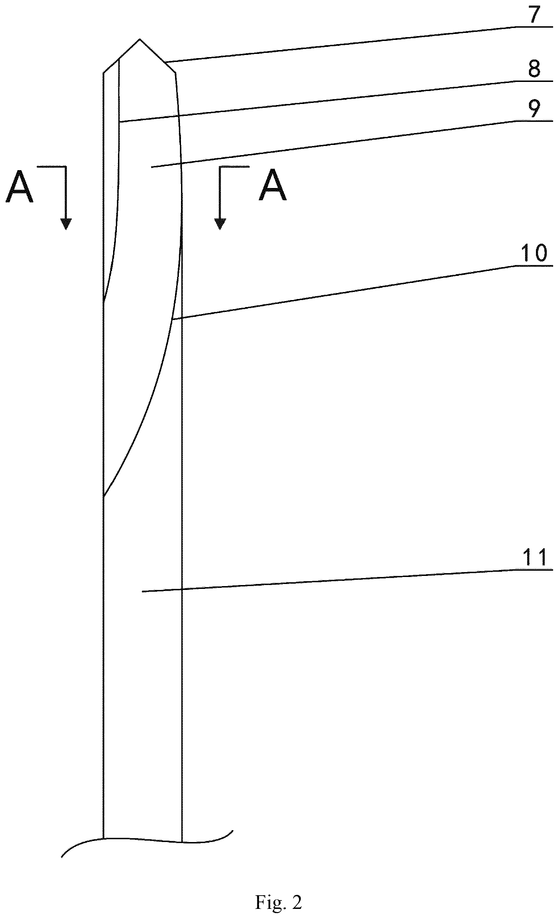

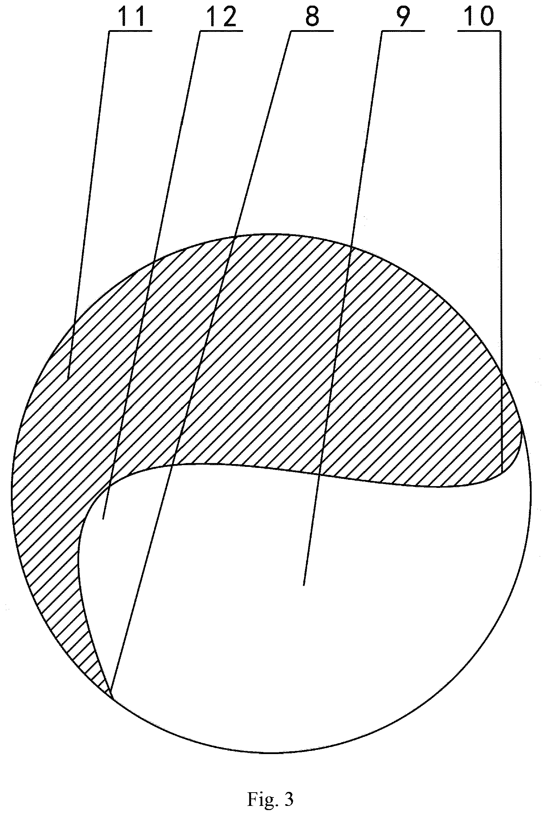

An electric spiral knife for eliminating neck humps, as shown in Fig. 1, 2 or 3, comprises a drill bit 1 and a rotary driver; the drill bit 1 comprises a rod body 11; a spiral groove 9 arranged clockwise along the side wall of the rod body 11 is installed at one end of the rod body 11; a conical end 7 is arranged at the end part of one end of the rod body 11; and a cutting edge 8 is located at the rear end of a notch of the cross section of the rod body 11 at the spiral groove 9 when the rod body 11 rotates clockwise according to the spiral groove 9 as the front end. A notch at the other side opposite to the cutting edge 8 is set as convex arc transition 10; the rotary driver comprises a motor 3; one end of a flexible shaft 2 is connected with a rotating shaft arranged on the motor 3; and the other end of the flexible shaft 2 is connected with the other end of the rod body 11. A chassis 4 is covered outside the motor 3. A foot switch 6 controls a switch of the motor 3, a connecting line 5 is arranged between the foot switch 6 and the motor 3, and the foot switch 6 is connected to a power supply. The motor 3 is communicated with one end of the flexible shaft 2 by a speed reducer. One spiral groove 9 is arranged on the rod body 11. Concave arc transition 12 is set between the cutting edge 8 and the spiral groove 9.

In implementation of the electric spiral knife for eliminating neck humps in the utility model, during use, the rod body 11 is placed on the outer edge of a base part of a neck hump parallel to the neck and back of a patient, so that the rod body 11 is perpendicular to the base part of the neck hump. A power supply is turned on; the foot switch 6 is trampled to start the motor 3; and the spiral groove 9 arranged at one end of the rod body 11 rotates in the clockwise direction. The utility model arranges one spiral groove 9 at one end of the rod body 11, which is matched with low speed outputted by the motor 3 added with the speed reducer to play a role of destroying the fibrous connective tissue while not destroying too many normal skin and tissue structures due to the cutting edge 8. After the skin of the patient is drilled by the cutting edge 8, the spiral groove 9 gradually extends into the base part of the neck hump, and the rod body 11 continues to rotate to make the spiral groove 9 move in a small range at the base part of the neck hump, so as to destroy the fibrous connective tissue at the base part of the neck hump. After the fibrous connective tissue at the base part of the neck hump is broken by the cutting edge 8, the rod body 11 is pulled out from the base part of the neck hump; the foot switch 6 is released; and a medical liposuction device is inserted into the neck hump along the hole drilled by the utility model for extracting the fat and the broken fibrous connective tissue in the neck hump to complete the operation.

The undetailed part of the utility model is the prior art.

## Claims

1. An electric spiral knife for eliminating neck humps, comprising a drill bit (1) and a rotary driver, wherein the drill bit (1) comprises a rod body (11); a spiral groove (9) arranged clockwise along the side wall of the rod body (11) is installed at one end of the rod body (11); a cutting edge (8) is located at the rear end of a notch of the cross section of the rod body (11) at the spiral groove (9) when the rod body (11) rotates clockwise according to the spiral groove (9) as the front end; a notch at the other side opposite to the cutting edge (8) is set as convex arc transition (10); the rotary driver comprises a motor (3); one end of a flexible shaft (2) is connected with a rotating shaft arranged on the motor (3); and the other end of the flexible shaft (2) is connected with the other end of the rod body (11).

2. The electric spiral knife for eliminating neck humps according to claim 1, wherein a chassis (4) is covered outside the motor (3).

3. The electric spiral knife for eliminating neck humps according to claim 1, wherein a foot switch (6) controls a switch of the motor (3), a connecting line (5) is arranged between the foot switch (6) and the motor (3), and the foot switch (6) is connected to a power supply.

4. The electric spiral knife for eliminating neck humps according to claim 1, wherein the motor (3) is communicated with one end of the flexible shaft (2) by a speed reducer.

5. The electric spiral knife for eliminating neck humps according to claim 1, wherein one spiral groove (9) is arranged on the rod body (11).

6. The electric spiral knife for eliminating neck humps according to claim 1, wherein concave arc transition (12) is set between the cutting edge (8) and the spiral groove (9).

7. The electric spiral knife for eliminating neck humps according to claim 1, wherein a conical end (7) is arranged at the end part of one end of the rod body (11).

8. The electric spiral knife for eliminating neck humps according to claim 1, wherein the rod body (11) is made of metal.
